(19) Europäisches Patentamt
European Patent Office
Office européen des brevets

(11) **EP 4 402 648 B1**

(12) **FASCICULE DE BREVET EUROPEEN**

(45) Date de publication et mention
de la délivrance du brevet:
**04.12.2024 Bulletin 2024/49**

(21) Numéro de dépôt: **23790701.9**

(22) Date de dépôt: **23.10.2023**

(51) Classification Internationale des Brevets (IPC):
**G06V 10/62** (2022.01)   **A61B 3/00** (2006.01)
**G06V 40/18** (2022.01)   **G06V 10/74** (2022.01)
**A61B 3/113** (2006.01)

(52) Classification Coopérative des Brevets (CPC):
**G06V 10/62; A61B 3/113; G06V 10/761;
G06V 40/18**

(86) Numéro de dépôt international:
**PCT/EP2023/079510**

(87) Numéro de publication internationale:
**WO 2024/088981 (02.05.2024 Gazette 2024/18)**

(54) **PROCÉDÉ DE DÉTECTION D'UN MOUVEMENT D'UN CORPS**

PROCÉDÉ DE DÉTECTION D'UN MOUVEMENT D'UN CORPS

METHOD FOR DETECTING A MOVEMENT OF A BODY

(84) Etats contractants désignés:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priorité: **24.10.2022   BE 202205859**

(43) Date de publication de la demande:
**24.07.2024   Bulletin 2024/30**

(73) Titulaire: **P³Lab**
**1341 Ceroux-Mousty (BE)**

(72) Inventeurs:
• **DAYE, Pierre Martin Jack Gérard**
**1420 Braine-l'Alleud (BE)**
• **WOINE, Rémy**
**5640 Graux (BE)**

(74) Mandataire: **Gevers Patents**
**De Kleetlaan 7A**
**1831 Diegem (BE)**

(56) Documents cités:
• **MALIK KRYSTYNA ET AL: "Eye blink detection
using Local Binary Patterns", 2014
INTERNATIONAL CONFERENCE ON
MULTIMEDIA COMPUTING AND SYSTEMS
(ICMCS), IEEE, 14 April 2014 (2014-04-14), pages
385 - 390, XP032651511, DOI: 10.1109/
ICMCS.2014.6911268**
• **LARRAZABAL A J ET AL: "Video-oculography
eye tracking towards clinical applications: A
review", COMPUTERS IN BIOLOGY AND
MEDICINE, vol. 108, 1 May 2019 (2019-05-01),
pages 57 - 66, XP085691894, ISSN: 0010-4825,
DOI: 10.1016/J.COMPBIOMED.2019.03.025**
• **VIDA ESMAEILI ET AL: "Spotting
micro-movements in image sequence by
introducing intelligent cubic-LBP", IET IMAGE
PROCESSING, IET, UK, vol. 16, no. 14, 25 July
2022 (2022-07-25), pages 3814 - 3830,
XP006117761, ISSN: 1751-9659, DOI:
10.1049/IPR2.12596**

**Description**

**Domaine technique**

La présente invention concerne :

- un procédé de détection d'un mouvement d'un corps,
- une méthode de suivi oculaire,
- une utilisation de la méthode pour détecter une saccade oculaire,
- un système informatique de traitement de données,
- un dispositif de détection d'un mouvement d'un oeil d'un patient,
- un programme d'ordinateur, et
- un support informatique lisible par un ordinateur.

**Art antérieur**

Aux fins d'évaluations neurologiques d'un patient, il est connu de soumettre ses yeux à des stimuli visuels et de suivre la réaction de ceux-ci à ces stimuli. Ces stimuli sont par exemple présentés sous la forme d'un ou plusieurs pixels sur un écran. Une ou plusieurs caméras capturent une séquence d'images des yeux du patient simultanément à la présentation de ces stimuli. Cette séquence d'images est ensuite traitée pour suivre le mouvement des yeux du patient.

Les saccades définissent une classe connue de mouvements susceptibles d'être détectés. Une « saccade » est une redirection rapide de la ligne de vue d'un centre d'intérêt vers un autre. Il s'agit d'un mouvement oculaire très rapide qui peut atteindre une vitesse de 600 degré par seconde. Afin de détecter et de suivre efficacement une saccade, il est donc nécessaire que la vitesse d'acquisition de la caméra soit suffisamment élevée, par exemple, environ 800 images par seconde.

Le nombre d'images à traiter lors de cette évaluation neurologique est donc tout à fait considérable. Leur traitement requiert des outils de calculs de puissance élevée ainsi qu'un certain temps d'exécution.

Dans le cadre distinct de la détection de clignements d'un oeil, l'article « Eye Blink Detection Using Local Binary Patterns » de K. Malik et B. Smolka, ICMCS, IEEE, de 2014, divulgue un procédé de transformation d'images de l'oeil en motifs binaires locaux entre lesquels une distance est déterminée et ensuite régularisée. Le signal ainsi obtenu comprend des pics qui sont connus comme correspondant aux clignements de l'œil recherchés. Bien que ce procédé convienne dans le cas d'un clignement d'un oeil, il ne permet pas une détection et un suivi efficace d'une saccade.

**Exposé de l'invention**

Un objet de l'invention est de rendre un tel traitement plus efficace. Dans ce but, l'invention propose un procédé de détection d'un mouvement d'un corps, par exemple un oeil, à partir d'une séquence d'images de ce corps, le procédé étant mis en oeuvre par ordinateur et comprenant les étapes suivantes :

(i) déterminer une mesure de similarité de chaque paire d'images d'une séquence de paires d'images, la séquence de paires d'images étant induite par la séquence d'images et incluant chaque image de cette dernière ;
(ii) déterminer une fonction dérivée de la mesure de similarité sur la séquence de paires d'images, la fonction dérivée correspondant à une dérivée filtrée obtenue par combinaison avec un filtre passe-bas ;
(iii) extraire au moins une image de chaque paire d'images en laquelle la fonction dérivée est supérieure à un seuil strictement positif ;
(iv) détecter le mouvement du corps sur base des images extraites à l'étape (iii).

Ce procédé permet une détection plus rapide et efficace de mouvements du corps dans la séquence d'images, et donc, au final, un traitement plus efficace de ces images, en particulier lorsque celles-ci sont très nombreuses.

En effet, plutôt que de traiter toutes les images de la séquence aux fins de la détection d'un mouvement du corps, l'inventeur propose d'extraire les images de la séquence contenant de prime abord une information d'un mouvement du corps, et de détecter ce mouvement sur ces images. Ces images peuvent en outre être traitées, par exemple par segmentation, pour suivre ou analyser plus en détails ce mouvement. Ainsi, toutes les images ne sont pas à considérer à cet égard, ce qui améliore significativement l'efficacité de la détection du mouvement du corps, et plus généralement du traitement des images.

Ce procédé est notamment motivé, dans le contexte de l'art antérieur, par le fait qu'environ 80% des images capturées par la caméra ne présentent pas de variation de pixels par rapport à l'image qui précède dans la séquence d'images et donc ne correspondent pas à des mouvements de l'œil. Pour améliorer le suivi d'un mouvement de l'œil, il était donc souhaitable de ne pas traiter ces images-là mais uniquement les images à partir desquels un mouvement de l'œil pouvait être detecté, ce que permet de procédé selon l'invention. Bien entendu, celui-ci n'est pas limité à cette application. L'homme du métier comprendra qu'il peut être utilisé pour toute détection de mouvement d'un corps dans une séquence d'images. Au plus la proportion d'images sur lesquelles le mouvement à détecter est faible, au plus le procédé selon l'invention est avantageux.

Pour déterminer à partir de quelles images détecter le mouvement du corps, et donc quelles images extraire à l'étape (iii), le procédé inclut les étapes (i) et (ii) qui sont au coeur de l'invention.

Tout d'abord, à l'étape (i), une similarité est mesurée entre des images. Les images sont ordonnées par paires à cet effet. Cette mesure permet de déterminer un degré de similitude entre les deux images de chaque paire, typiquement pixel par pixel. Cette étape ne suffit toutefois pas à elle seule à déterminer les images à extraire à l'étape (iii). En effet, deux images peuvent présenter des variations plus ou moins importantes de pixels sans que le corps représenté sur les images n'ait subi de mouvement. Ces variations sont, par exemple, due à du bruit et/ou à une luminosité variable de l'environnement du corps lors de l'acquisition des images. L'étape (ii) vise à dériver la mesure de similitude utilisée à l'étape (i) pour éliminer la composante statique des images en terme de pixels et mieux distinguer un réel mouvement du corps des variations susmentionnées. Ainsi, l'extraction de l'étape (iii) est plus fine et répond davantage aux besoins d'efficacité recherchée dans ce contexte.

Le seuil utilisé à l'étape (iii) permet avantageusement d'adapter la proportion des images extraites. Il correspond à un niveau de compression de la séquence d'images. Un seuil nul n'est bien évidemment pas souhaitable car il correspondrait à conserver toutes les images indépendamment de leur représentation. Le seuil est fixé selon le type de mesure de similarité et de fonction dérivée utilisée.

La détection du mouvement du corps peut ensuite se faire à l'étape (iv) par traitement des images extraites. Dans un mode de réalisation, la détection prend la forme d'une simple preuve de l'existence d'un mouvement du corps qui ressort à première vue des images de par l'étape (iii). Un traitement des images extraites peut être effectué lors de l'étape (iv) pour détecter et/ou caractériser de façon plus précise le mouvement du corps tel que connu de l'homme du métier, l'essentiel restant que cette étape est effectuée sur un nombre réduit d'images.

De par l'usage des termes « mis en oeuvre par ordinateur », le procédé de l'invention implique l'utilisation d'un ordinateur, d'un réseau informatique et/ou de n'importe quel autre appareil programmable (par exemple, un smartphone, une tablette, un FPGA, ...) ou programmé (par exemple, un circuit intégré / ASIC, ...). En particulier, le terme « ordinateur » ne peut être interprété de façon restrictive. Les étapes de détermination relèvent donc au moins partiellement d'un caractère informatique sous-jacent. Par exemple, une ou plusieurs de ces étapes peuvent constituer en une détermination par calcul algorithmique.

Dans le cadre de ce document, le terme « corps » est utilisé génériquement et peut se référer tant à un objet physique qu'à tout ou partie d'un corps humain.

Dans le cadre de ce document, les images sont préférentiellement de même taille, par exemple 640 x 480 pixels. Le procédé de l'invention peut cependant être appliqué sélectivement à un sous-ensemble des pixels de l'image comme détaillé ci-après. Dans le cadre de ce document, les images sont de préférence capturées selon une même fréquence, par exemple, à 797 images par seconde.

Le terme « séquence » implique de lui-même une notion d'ordre entre les éléments de la séquence. De préférence, l'ordre associé à la séquence d'images est celui dans lequel les images ont été capturées, par exemple par une caméra vidéo. Par exemple, dans ce cas, la séquence d'images forme une suite d'images dans le temps, représentant, par exemple, un mouvement d'une partie d'un corps humain, par exemple d'un oeil. D'autres notions d'ordre peuvent être utilisées.

Les paires d'images de l'étape (i) sont également ordonnées vu qu'elles sont classées en séquence. Cette séquence est « induite » par la séquence d'images, c'est-à-dire que la notion d'ordre sur les paires est induite par celle sur les images. En d'autres termes, une première paire $\{I_1, I_2\}$ précède une deuxième paire $\{I_1', I_2'\}$ dans la séquence des paires, avec $I_1$ et $I_1'$ précédant respectivement $I_2$ et $I_2'$ dans la séquence d'images, si et seulement si, dans la séquence d'images :

- $I_1$ précède ou correspond à $I_1'$ et
- $I_2$ précède ou correspond à $I_2'$.

Les images d'une même paire sont évidemment de préférence distinctes. Toutes les images de la séquence d'images se retrouvent dans au moins une des paires d'images. La similitude entre les images de la séquence d'images peut ainsi être mesurées successivement selon l'ordre induit par la séquence d'images.

Deux exemples préférés de séquences de paires d'images sont fournis :

- selon une première réalisation, la séquence de paires d'images comprend (et de préférence consiste en) chaque paire de deux images consécutives dans la séquence d'images ;

- selon une deuxième réalisation, la séquence de paires d'images comprend (et de préférence consiste en) chaque paire formée de la première image de la séquence d'images et d'une autre image de la séquence d'images.

En d'autres termes, si la séquence d'images correspond à

$$(I_n)_{1 \le n \le N} = (I_1, I_2, I_3, \ldots, I_N)$$

où N désigne le nombre d'images lesdites première et deuxième réalisations correspondent de façon respective aux séquences de paires

$$(\{I_n, I_{n+1}\})_{1 \le n \le N-1} \quad \text{et} \quad (\{I_1, I_n\})_{2 \le n \le N}$$

Dans la première réalisation, la similitude entre les images est mesurée de façon progressive par deux images consécutives sur toute la séquence d'images (image 1 avec image 2, puis image 2 avec image 3, et ainsi de suite). Ainsi, il est possible de détecter très précisément une absence de similitude entre deux images qui se suivent dans la séquence d'images. Dans la deuxième réalisation, la similitude est mesurée par rapport à la première image, de sorte qu'il est possible de déterminer à partir de quelle image un mouvement du corps pourrait être détecté.

L'usage, dans ce document, du verbe « comprendre », de ses variantes ou conjugaisons, n'exclut pas la présence d'éléments autres que ceux mentionnés. De façon semblable, l'usage de l'article indéfini « un », « une », ou de l'article défini « le », « la » ou « l' », pour introduire un élément n'exclut pas la présence d'une pluralité de ces éléments.

L'utilisation de « mesures de similarité » pour quantifier une différence entre les pixels de deux images est connue de l'homme du métier. Il existe notamment plusieurs telles mesures qui peuvent être utilisées dans le cadre de l'invention, par exemple par une mesure corrélation. Toutefois, dans le cadre de l'invention, une mesure de similarité préférée avantageuse des paires d'images est identifiée. Elle correspond à un coefficient de Bhattacharyya (noté « BC ») de deux distributions de probabilités discrètes associées aux images de la paire d'images et définies à partir de pixels de ces images.

De façon précise, si p et q désignent les distributions de probabilité définies sur un ensemble P des pixels des deux images de la paire d'images, le coefficient de Bhattacharyya est défini par :

$$BC(p, q) = \sum_{x \epsilon P} \sqrt{p(x)\, q(x)} \, .$$

L'ensemble de pixels peut correspondre à la totalité des pixels de l'image ou à un sous-ensemble de ceux-ci, comme par exemple le fait de ne sélectionner qu'une ligne et/ou une colonne sur deux. Même si c'est en général le logarithme népérien de l'inverse de ce coefficient qui est utilisé comme mesure de similarité, dans le cadre de l'invention, c'est bien le coefficient lui-même qui est utilisé, notamment aux fins de l'exécution de l'étape (ii).

Il existe aussi plusieurs façons connues de l'homme du métier d'associer une distribution de probabilité aux pixels d'une image pour évaluer ce coefficient. Dans un mode de réalisation de l'invention, la distribution de probabilité p discrète associée à chaque image est définie en chaque pixel x de l'image par :

$$p(x) = \frac{G(x)}{\sum_{y \epsilon P} G(y)}$$

où G désigne une fonction de niveau de gris définie sur les pixels de l'image. En d'autres termes, p(x) est définie comme un quotient d'un niveau de gris en le pixel p par une somme de niveaux de gris en chaque pixel de l'image. Le niveau de gris en un pixel est typiquement évalué de façon numérique et discrétisée, par exemple sur une échelle d'entiers allant de 0 à 255.

Avantageusement, l'utilisation du coefficient de Bhattacharyya par rapport à d'autres mesures de similarité est indépendante de la luminosité des images, ce qui rend le procédé selon ce mode de réalisation plus robuste dès l'étape (i), en particulier en vue de détecter spécifiquement des mouvements du corps.

L'étape (ii) peut également être implémentée de différente façon au moyen de plusieurs dérivées discrètes connues de l'homme du métier. En particulier, le terme « fonction dérivée » est utilisé de façon équivalente à « dérivée » dans ce document, tel que le comprendrait un homme du métier. Au vu du domaine discret pour lequel la dérivée est déterminée, il s'agit d'une dérivée filtrée En particulier, un filtre passe-bas est composé avec la dérivation de la mesure de similarité dans le but d'éliminer ou de réduire le bruit éventuel sur les images. La fonction dérivée correspond donc à une dérivée filtrée obtenue par combinaison avec un filtre passe-bas.

La dérivée filtrée correspond de préférence à une discrétisation, bilinéaire de façon préférée, d'une transformée de Laplace de fonction de transfert donnée par H(s) = s (s $\tau$+1)$^{-1}$ où $\tau$ est une constante (plus précisément, constante de temps du filtre passe-bas combiné à la dérivation). Cette discrétisation permet l'utilisation de cette expression régulière dans un système digital. Le fait que la discrétisation soit bilinéaire est préféré et avantageux afin de garantir une stabilité adéquate du filtre passe-bas entre sa forme régulière donnée par la fonction de transfert et sa forme discrétisée.

De façon concrète, si $S_n$ correspond à la mesure de similarité de la n-ième paire d'images de la séquence de paires d'images, si $S'_n$ correspond à l'évaluation de la fonction dérivée de la mesure de similarité sur cette n-ième paire d'images, et si t désigne une durée constante entre deux images capturées par une caméra, la dérivée filtrée selon ce mode de réalisation est définie récursivement par

$$ S'_n = 2\left(\frac{S_n - S_{n-1}}{2\tau + t}\right) + \left(\frac{2\tau - t}{2\tau + t}\right)S'_{n-1}. $$

Le pas initial $S'_1 = S_1$ est typiquement utilisé. La constante $\tau$ est un nombre réel de préférence un multiple entier de la période d'échantillonnage t, ce multiple étant, par exemple, compris entre 2 et 10, par exemple 5.

En revenant à l'énoncé général du procédé selon l'invention, il peut être noté que l'application (ou évaluation) de la fonction dérivée de la mesure de similarité sur la séquence de paires d'images fournit de façon naturelle une distribution de données. De préférence, le seuil utilisé à l'étape (iii) est défini par un percentile de cette distribution. Il s'agit d'une façon avantageusement pratique de compresser la séquence d'images adéquatement en en extrayant un pourcentage spécifique des images selon l'objet de la détection de mouvement. De préférence, ce percentile est choisi entre le quatre-vingtième et le nonante-neuvième percentile, ce qui rend le traitement des images sensiblement plus efficace vu que moins d'une image sur cinq est extraite de la séquence d'image. Dans le cas où le procédé vise à la détection de saccade oculaire, il a été constaté par l'inventeur que l'utilisation du nonante-cinquième percentile fournissait des résultats tout à fait satisfaisant car les saccades sont des mouvements très brefs et rapides. Dans le cas particulier de la dérivée filtrée du coefficient de Bhattacharyya basé sur un niveau de gris en les pixels des images, pour $\tau$ = 5t et t = 3,75 ms, il a été mis en évidence que le seuil correspondant de 0,00175 permettait avantageusement de détecter de façon efficace toutes les saccades oculaire de tout patient, en extrayant au plus 5% des images de la séquence d'images, quelle que soient les conditions d'exécution du procédé.

Selon un mode de réalisation préféré de l'invention, l'image qui est extraite d'une paire à l'étape (iii) comprend l'image de rang le plus élevé dans la séquence d'images parmi les deux images de la paire d'images. Ceci permet de conserver pour l'étape (iv) et/ou le traitement éventuel ultérieure les images en lesquels il se « passe quelque chose », en d'autres termes, les images où un début de mouvement est susceptible d'être détecté. Dans le cas d'une paire de la forme {$I_n$, $I_{n+1}$}, l'image $I_{n+1}$ est donc extraite, et dans le cas d'une paire de la forme {$I_1$, $I_n$}, l'image $I_n$ est extraite, si l'évaluation de la fonction dérivée de la mesure de similarité en cette paire est supérieure au seuil.

Les deux images de la paire peuvent optionnellement être extraites.

La détection du mouvement du corps peut être faite à l'étape (iv) sur la seule base des images extraites. Toutefois, d'autres images peuvent être utilisées, par exemple, pour des raisons de sécurité ou de fiabilité du procédé. Ainsi, selon une réalisation préférée de l'invention, l'étape (iii) comprend également une extraction périodique d'une image de la séquence d'images. Cette extraction peut se faire à une fréquence comprise entre 20 et 40 Hz, par exemple 30 Hz, pour une vitesse d'acquisition usuelle des images par une caméra. De façon alternative, une image toute les 20 à 40 images, par exemple toute les 30 images, peut être extraite. Vu que la proportion d'images supplémentaires ainsi extraites est faible, la prise en compte de ces images n'affectent pas significativement l'efficacité du procédé tout en permettant d'en renforcer avantageusement la fiabilité et la robustesse.

De préférence, le procédé comprend aussi une étape de segmentation des images extraites à l'étape (iii). Cette étape de segmentation peut intervenir lors de l'étape (iv) et/ou simultanément ou postérieurement à celle-ci. Elle peut faire partie d'un « traitement » ultérieure des images extraites, par exemple, afin de classifier, étudier et/ou quantifier les mouvements du corps détectés. La segmentation en tant que telle est un procédé connu de l'homme du métier. Elle peut être de tout type connu. Elle peut, par exemple, être fondée sur des régions ou des contours au niveau des images de la séquence, et/ou aussi sur un classement des pixels par intensité (par exemple, lumineuse, de niveau de gris, etc.). La segmentation peut se faire localement, sur une zone définie des images, ou globalement.

De préférence, dans le cas où la segmentation de chaque image extraite à l'étape (iii) est définie par une fonction de segmentation de l'image, cette étape de segmentation est mise en oeuvre via les sous-étapes suivantes :

(s1) déterminer une estimation de la fonction de segmentation de chaque image extraite à l'étape (iii) ;

(s2) déterminer récursivement la fonction de segmentation de chaque image extraite à l'étape (iii) à partir de son estimation.

Grâce à cette implémentation, il est rendu possible de segmenter de façon à la fois rapide et efficace les images extraites à l'étape (iii). En effet, les fonctions de segmentation sont déterminées récursivement, et donc déduites les unes des autres. L'ordre associé à la récursion est typiquement celui induit par la séquence d'images. Ceci permet d'éviter un traitement individuel des images, donc plus long et moins efficace. Cet avantage est accru car chaque fonction de segmentation est déterminée à partir de son d'estimation (qui peut, elle-même, être déterminée récursivement ou non selon le cas), constituant ainsi une étape intermédiaire plus simple à exécuter que la segmentation directe des images. Moins de calculs sont ainsi nécessaires car la détermination d'une fonction de segmentation s'apparente à l'ajustement d'un résidu par rapport à son estimation. L'aspect récursif accroit également la fiabilité de la détermination des fonctions de segmentation d'images car celle-ci ne repose alors pas uniquement sur les estimations mais aussi sur les fonctions déjà déterminées.

Le terme « fonction de segmentation » utilisé ci-dessus fait référence à une fonction définie sur les pixels des images associant un modèle et/ou une structure des pixels des images, typiquement les pixels ayant une image « non nulle » (ou non constante) par la fonction. Lorsqu'un tel modèle ou une telle structure peut être mis en évidence au niveau de pixels d'une première des images extraites à l'étape (iii) (de façon algorithmique ou manuelle), l'étape de segmentation permet d'intégrer de façon avantageuse et récursive ce modèle ou cette structure sur les autres images extraites à l'étape (iii) pour suivre et/ou analyser son évolution et/ou le détecter efficacement, sans traiter chaque image indépendamment.

Les notations (s1) et (s2) ne doivent pas être interprétées comme le fait que la sous-étape (s1) soit exécutée intégralement avant la sous-étape (s2). Celles-ci peuvent être imbriquées, comme dans le mode de réalisation préféré suivant.

De préférence, l'estimation d'une fonction de segmentation d'une deuxième image est déterminée à la sous-étape (s1) par une composition d'un champ de vecteurs avec une fonction de segmentation d'une première image, la première image précédant la deuxième image dans la séquence d'images, la fonction de segmentation de la première image étant déterminée au préalable à la sous-étape (s2), et le champ de vecteurs correspondant en un déplacement de pixels entre la deuxième et la première images. De préférence, la première image précède de façon directe la deuxième image dans la séquence des images extraites à l'étape (iii). De préférence, chaque estimation de fonction de segmentation d'une n-ième image est déterminée de façon semblable à partir de la fonction de segmentation de la (n-1)-ième image de la séquence des images extraites à l'étape (iii). De plus, les termes « première » et « deuxième » ne doivent pas être interprété ci-dessus comme étant limité à la position de ces images selon l'ordre induit sur les images extraites à l'étape (iii) par l'ordre correspondant à la séquence d'image.

Le champ de vecteurs correspond à une forme de comparaison entre deux images et encode un déplacement que les pixels de la deuxième image devraient subir pour revenir à une position associée à la première image. De préférence, il est calculé par flux optique.

Les estimations des fonctions de segmentation, en tant qu'approximation au premier ordre de ces fonctions, sont ainsi aisément calculables, ce qui améliore encore l'efficacité et la rapidité d'exécution de l'étape de segmentation, et donc du procédé la comprenant selon l'invention. En outre, dans le cas d'images d'un oeil aux fins d'un suivi oculaire, ce mode de réalisation permet de déduire aisément une estimation précise de la position de l'œil ou d'une partie de l'œil à suivre sur chaque image extraite à l'étape (iii) et de limiter ainsi fortement une zone d'intérêt de l'image sur laquelle identifier l'œil ou la partie de l'œil via l'étape (s2). La zone est d'autant plus limitée, et donc l'estimation fiable, que l'œil ou la partie de l'œil à suivre varie de façon limitée en position entre deux images consécutives extraites à l'étape (iii), ce qui est généralement le cas lorsque la séquence d'images initiale comprend un grand nombre d'images capturées par seconde. En particulier, cette réalisation est tout à fait adaptée à la détection et l'étude des saccades oculaires.

L'invention propose également une avantageuse méthode de suivi oculaire comprenant les étapes suivantes :

(a) fournir une séquence d'images d'un oeil d'un patient ;

(b) détecter un mouvement de l'œil à partir de la séquence d'images ;

où l'étape (b) est mise en oeuvre au moyen du procédé selon l'invention. Dans ce cas, le procédé est appliqué dans le cas où le « corps » correspond à l'œil à partir de la séquence d'images fournie à l'étape (a). Comme il ressort de l'énoncé de la méthode, il est prévu un suivi oculaire sur base de la détection de l'étape (b).

La méthode selon l'invention permet un suivi oculaire plus efficace vu que le mouvement de l'œil est détecté à l'étape (b) plus efficacement et rapidement. De manière plus générale, les modes de réalisation et avantages du procédé selon l'invention se transposent *mutatis mutandis* à cette méthode de suivi oculaire.

De préférence, l'étape (b) est mise en oeuvre au moyen du procédé lorsqu'il comprend une étape de segmentation des images extraites à l'étape (iii). L'étape de segmentation susdite est alors préférentiellement mise en oeuvre au moins au voisinage d'une représentation de l'iris de l'œil sur chacune des images extraites à l'étape (iii), et la méthode comprend en outre les étapes suivantes :

(c) déterminer une position (ou information de position) d'un limbe de l'œil sur base de la segmentation des images extraites à l'étape (iii) ;
(d) déterminer une position (ou information de position) de l'œil sur base de la position (ou information de position) du limbe de l'œil déterminé à l'étape (c).

La méthode selon ce mode de réalisation permet d'obtenir un suivi oculaire rapide et précis par rapport aux méthodes connues de l'art antérieur. En effet, la segmentation des images extraites à l'étape (iii) est effectuée récursivement de sorte que chaque segmentation est déduite d'une estimation de segmentation et d'au moins une des segmentations précédentes, ce qui permet une exécution de l'étape de segmentation de l'étape (b) précise et rapide. La segmentation d'une image de l'œil au voisinage des pixels associés à l'iris de l'œil permet de mettre en évidence une forme elliptique dans le cas d'une segmentation de contour, correspondant essentiellement au limbe de l'œil, dont la position peut ainsi être avantageusement déduite à l'étape (c) selon les paramètres définissant l'ellipse.

En outre, la méthode selon ce mode de réalisation propose de tenir compte en premier lieu du limbe pour déterminer la position de l'œil à l'étape (d), et se distingue ainsi de la plupart des méthodes connues qui se basent distinctivement sur des données relatives à la pupille pour déterminer la position de l'œil. En effet, la détermination de la position de l'œil par ces méthodes est moins précise que le mode de réalisation de la méthode selon la présente invention. Ceci est dû au fait que la détermination de caractéristiques de position de la pupille est entachée de plusieurs erreurs dues, par exemple, au fait que la vitesse et l'accélération des mouvements oculaires (pouvant atteindre de façon respective 600°/s et 35000°/s$^2$ pendant des saccades) induit un mouvement relatif entre la pupille et le limbe de l'œil, ou encore au fait que toute tâche noire (par exemple, du mascara) est prise en compte dans les calculs par la plupart des suiveurs oculaires connus.

La position de l'œil peut toutefois être en outre déterminée à l'étape (d) sur base d'information de position de la pupille de l'œil préalablement déterminées à l'étape (c) sur base de la segmentation des images extraites à l'étape (iii), ce qui permet d'obtenir un suivi oculaire encore plus précis.

La rapidité et l'efficacité de la méthode de suivi oculaire la rende en outre particulièrement intéressant pour suivre un oeil sur un flux continu d'images vidéo. La séquence d'images initiale est alors fournie sous la forme de ce flux d'images, par exemple, au moyen d'une caméra pointé sur l'œil. Le sujet auquel appartient l'œil est typiquement stimulé à suivre une cible mobile sur un écran. La méthode de suivi oculaire permet alors une étude des mouvements de l'œil, par exemple, aux fins de détection de maladies neurodégénératives.

En particulier, et au regard des avantages de l'invention exposés ci-dessus, l'invention propose spécifiquement une utilisation de la méthode de suivi oculaire selon l'invention, pour détecter une saccade de l'œil, typiquement à partir d'un flux d'images vidéo de l'œil constituant la séquence d'images initiale.

De façon générale, les applications de la méthode de suivi oculaire ne sont pas limitées à ces exemples. L'homme du métier comprendra plus généralement que la méthode de suivi oculaire selon l'invention ne constitue qu'une application parmi d'autres du procédé selon l'invention. Celui-ci peut être utilisé dans nombre d'applications pratiques, telles que la surveillance des yeux d'un conducteur d'un véhicule, la reconnaissance faciale, par exemple dans des filtres de logiciels de vidéo-conférence, la détection de tumeurs dans des images, par exemple RX, via scan CT et/ou CBCT.

La présente invention propose également :

- un système (informatique) de traitement de données comprenant des moyens configurés pour mettre en oeuvre le procédé de détection de mouvement selon l'un quelconque des modes de réalisation de l'invention ;
- un programme d'ordinateur comprenant des instructions qui, lorsque le programme d'ordinateur est exécuté par un ordinateur, conduisent celui-ci à mettre en oeuvre le procédé selon l'un quelconque des modes de réalisation de l'invention ;
- un support (informatique) lisible par un ordinateur, et sur lequel est enregistré le programme d'ordinateur susdit.

Le système de traitement de données comprend, par exemple, au moins un hardware ou matériel informatique parmi :

- un ordinateur muni d'un processeur pour exécuter des algorithmes, de préférence d'une architecture de type PC ou d'une architecture de type embarqué (ARM) ;
- un circuit intégré ASIC spécifique à une application et/ou à un algorithme ;
- un circuit intégré (logique) du type FPGA, qui est typiquement reconfigurable ou reprogrammable après fabrication.

Le système de traitement de données consiste plus préférentiellement en un système embarqué. Ce mode de réalisation est avantageusement rendu possible car les étapes (i) à (iii) du procédé selon l'invention nécessite une faible puissance de calcul, mais aussi et surtout car elles permettent de réduire significativement le nombre de calculs et la puissance de calcul nécessaires à l'exécution des étapes ultérieures du procédé, en particulier lorsque celles-ci comprennent un traitement des images extraites à l'étape (iii). Ledit système embarqué est typiquement muni d'un circuit intégré du type FPGA.

Le support informatique lisible par ordinateur consiste de préférence en au moins un support informatique (ou un ensemble de tels supports) apte à stocker des informations numériques. Il comprend, par exemple, au moins un parmi : une mémoire numérique, un serveur, une clé USB, un ordinateur. Il peut être dans un nuage de données (cloud).

Les modes de réalisation et les avantages du procédé selon l'invention se transposent *mutatis mutandis* au système informatique de traitement de données, au programme d'ordinateur et au support informatique lisible par un ordinateur selon l'invention.

L'invention propose aussi un dispositif de détection d'un mouvement d'un oeil d'un patient comprenant une chambre de vision pour le patient comprenant, en une première extrémité, un pourtour d'ouverture apte à être en contact avec une partie de tête du patient, et en une deuxième extrémité, un écran d'affichage de stimuli visuels. L'écran est intégré dans une unité informatique comprenant :

- le système informatique selon l'invention, typiquement un système embarqué muni d'un circuit intégré du type FPGA ;
- au moins une caméra d'acquisition d'images de l'œil dirigée vers la première extrémité et couplée électroniquement au système informatique pour lui transmettre une séquence d'images de l'œil.

Le patient place typiquement sa tête sur le pourtour d'ouverture de façon à porter son regard dans la chambre de vision, et en particulier vers la deuxième extrémité au sein de laquelle est agencée l'écran. Le pourtour d'ouverture est par exemple en contact avec le front et le nez du patient. La chambre de vision fournit ainsi un environnement mi-clos isolé des perturbations extérieures et adapté à la détection et/ou au suivi du mouvement d'un oeil ou des yeux du patient soumis aux stimuli visuels, par exemple aux fins d'une évaluation neurologique. De préférence, le dispositif comprend au moins une première telle caméra dirigée vers un premier oeil du patient et au moins une deuxième telle caméra dirigée vers un deuxième oeil du patient pour détecter et/ou suivre le mouvement des deux yeux du patient.

Comme le dispositif comprend ledit système embarqué, il est rendu possible d'implémenter le procédé et/ou la méthode selon l'invention en temps réel sur le flux d'images vidéo acquises par la caméra. Cette technologie embarquée rend le dispositif selon l'invention performant pour détecter et/ou suivre un mouvement de l'œil ou des yeux d'un patient.

La chambre de vision comprend typiquement une paroi latérale s'étendant autour d'un axe, entre les première et deuxième extrémités. De préférence, une distance entre les première et deuxième extrémités est réglable à une longueur fixe de cette paroi latérale, mesurée le long de l'axe, parmi différentes longueurs possibles, celles-ci étant obtenues par fixations amovibles successives d'une ou plusieurs portions amovibles de paroi latérale à une portion fixe de paroi latérale s'étendant à partir de la première extrémité.

Ce mode de réalisation du dispositif permet de modifier la distance entre les première et deuxième extrémités, donc entre l'œil du patient et l'écran, lorsque le dispositif est en fonctionnement, par exemple, lors d'une évaluation neurologique du patient, afin de pouvoir détecter et/ou suivre le mouvement de l'œil suivant plusieurs conditions d'éloignement par rapport aux stimuli visuels. En effet, pour modifier cette distance, il est suffisant de fixer ou d'enlever une ou plusieurs des portions amovibles, ce qui est particulièrement aisé. Lesdites fixations amovibles fonctionnent notamment à l'image d'un empilement de briques de LEGO ®. Le fait que chaque longueur soit fixe permet une calibration efficace du dispositif avant son usage mais surtout une bonne utilisation du dispositif dans un environnement normalisé de dimensions connues.

Selon un mode de réalisation du dispositif de l'invention, l'unité informatique comprend en outre une horloge centrale configurée pour commander :

- un affichage des stimuli visuels, au moyen de l'écran, par un rafraîchissement de pixels de l'écran à une première fréquence ;
- une acquisition des images de l'œil, au moyen de la caméra, à une deuxième fréquence ;

les première et deuxième fréquences étant égales ou multiples l'une de l'autre par un nombre naturel non nul.

Ce mode de réalisation implémenté au niveau de l'unité informatique permet avantageusement de détecter et/ou de suivre de façon plus précise le mouvement de l'œil du patient. En effet, il est possible de déterminer précisément à quel(s) pixel(s) rafraîchi(s) de l'écran correspond une image capturée par la caméra. Il y a donc une certitude temporelle dans la correspondance des données fournies par l'écran et la caméra car l'horloge dont est pourvu l'unité informatique commande, à elle seule, la valeur des première et deuxième fréquences. En d'autres termes, l'horloge centrale commande le déclenchement du rafraîchissement des pixels de l'écran et la capture des images par le caméra de façon synchronisée. Le choix des valeurs de fréquences comme étant multiples entières l'une de l'autre permet de conserver la relation temporelle entre l'acquisition des images par la caméra et la présentation des stimuli visuels par l'écran.

**Brève description des figures**

D'autres caractéristiques et avantages de la présente invention apparaîtront à la lecture de la description détaillée qui suit pour la compréhension de laquelle on se reportera aux figures annexées parmi lesquelles :

- la figure 1 illustre un exemple de représentation graphique d'une détermination d'une mesure de similarité selon l'étape (i) du procédé de l'invention ;
- la figure 2 illustre un exemple de représentation graphique d'une détermination d'une fonction dérivée de la mesure de similarité de la figure 1 selon l'étape (ii) du procédé de l'invention ;
- les figures 3A et 3B illustrent deux vues schématiques tridimensionnelles d'une chambre de vision d'un mode de réalisation du dispositif selon l'invention ;
- la figure 4 illustre une vue schématique d'une unité informatique d'une chambre de vision d'un mode de réalisation du dispositif selon l'invention.

Les dessins des figures ne sont pas à l'échelle. Des éléments semblables sont en général dénotés par des références semblables dans les figures. Dans le cadre du présent document, les éléments identiques ou analogues peuvent porter les mêmes références. En outre, la présence de numéros ou lettres de référence aux dessins ne peut être considérée comme limitative, y compris lorsque ces numéros ou lettres sont indiqués dans les revendications.

**Description de modes de réalisation de l'invention**

Cette partie présente une description de modes de réalisation préférés de la présente invention. Cette dernière est décrite avec des réalisations particulières et des références à des figures mais l'invention n'est pas limitée par celles-ci. En particulier, les dessins ou figures décrits ci-dessous ne sont que schématiques et ne sont pas limitants.

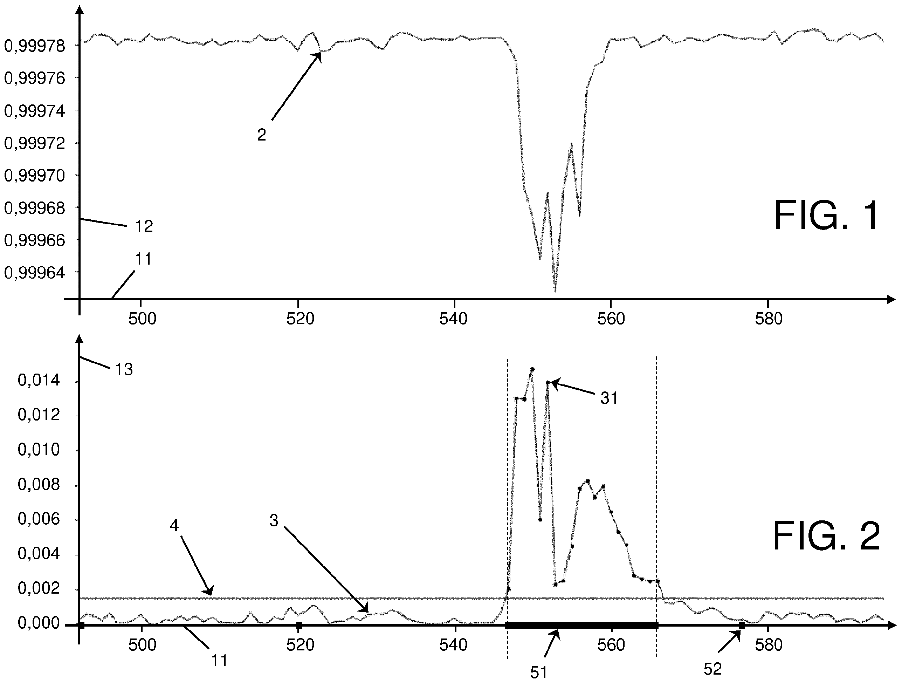

Les figures 1 et 2 illustrent un exemple concret d'exécution des étapes (i) à (iii) du procédé de l'invention sur une séquence d'images vidéo d'un oeil capturées par une caméra. Les images sont capturées par la caméra à 3,75 ms d'intervalle. Elles sont associées chacune à une distribution de probabilité discrète définie en un pixel de l'image comme le rapport entre le niveau de gris en ce pixel évalué de façon discrétisée sur une échelle de 0 à 255 et la somme des niveaux de gris en chaque pixel de l'image évalués de la même façon.

La séquence de paires d'images considérées dans l'exécution de l'étape (i) consiste en chaque paire formée par deux images consécutives dans la séquence d'images. La mesure de similarité de chaque paire d'images qui est considérée est celle définie par le coefficient de Bhattacharyya (BC) des deux distributions de probabilités discrètes associées aux deux images de la paire d'images.

La figure 1 représente un graphe de l'évolution de cette mesure de similarité 2. La référence 2 est utilisée à la fois pour désigner la mesure de similarité et son graphe. L'axe 11 indique un numéro d'ordre des images dans la séquence. Plus précisément, chaque numéro correspond au numéro de l'image ayant le rang le plus élevé dans une paire d'images, et comme la séquence des paires d'images est formées par une succession de deux images consécutives, une numérotation des paires d'images revient à celle des images dans la séquence initiale. L'axe 12 est celui sur lequel est lue valeur du coefficient de Bhattacharyya de chaque paire d'images.

Afin d'éliminer la composante statique du graphe de la figure 1 et d'éliminer la dépendance du coefficient de Bhattacharyya du bruit éventuel des images, il est utilisé une fonction dérivée 3 de la mesure de similarité 2 sur la séquence de paires d'images, conformément à l'étape (ii) du procédé, combiné avec un filtre passe-bas. De façon plus précise et préférée, une dérivée filtrée est appliquée à la mesure de similarité 2.

La figure 2 représente un graphe de l'évolution d'une telle fonction dérivée 3. La référence 3 est ici utilisée à la fois pour désigner la fonction dérivée et son graphe. La dérivée filtrée considérée pour cet exemple est plus précisément une discrétisation bilinéaire de la transformée de Laplace de fonction de transfert $H(s) = s \, (s\tau+1)^{-1}$ où $\tau$ est une constante de temps correspondant à cinq fois l'intervalle de temps entre la capture de deux images, i.e. 18,75 ms. Cet exemple de dérivée filtrée est introduit dans l'exposé de l'invention, notamment sous forme explicite et discrétisée. La valeur de la fonction dérivée 3 en chaque paire d'images peut être lue sur l'axe 13.

Comme le constatera aisément l'homme du métier à la lumière des figures 1 et 2, l'application de la fonction dérivée 3 à la mesure de similarité 2 permet de mettre en lumière les paires d'images consécutives en lesquelles un changement significatif en terme de pixels a eu lieu.

Les points du graphe de la fonction dérivée 3 sont filtrés pour ne garder que ceux 31 ayant une valeur lue sur l'axe 13 au-dessus d'un seuil 4 strictement positif tel qu'illustré en figure 2. Le seuil de 0,00175 introduit dans l'exposé de l'invention est utilisé à cet effet. L'étape (iii) du procédé est alors exécutée et correspond à l'extraction de l'image 51 de rang le plus élevé parmi les images de chaque paire d'images associée à un point 31 du graphe. Ceci permet d'une part de détecter un mouvement clair de l'œil, environ entre les images 545 et 564, sans analyser les images une à une. Il est ainsi possible d'analyser ce mouvement plus précisément en traitant ultérieurement ces images 51, par exemple via l'étape de segmentation et/ou les étapes (c) et (d) de la méthode de suivi oculaire décrites dans l'exposé de l'invention, en particulier pour détecter des saccades oculaires, ce qui rend une telle analyse plus efficace et rapide et sans nécessiter une grande puissance de calcul. Le procédé peut en outre être renforcé par la sélection automatique, à une certaine fréquence, par exemple 30 Hz, d'images 52 de la séquence d'images qui seront considérées, avec les images 51, lors d'un traitement éventuel ultérieur.

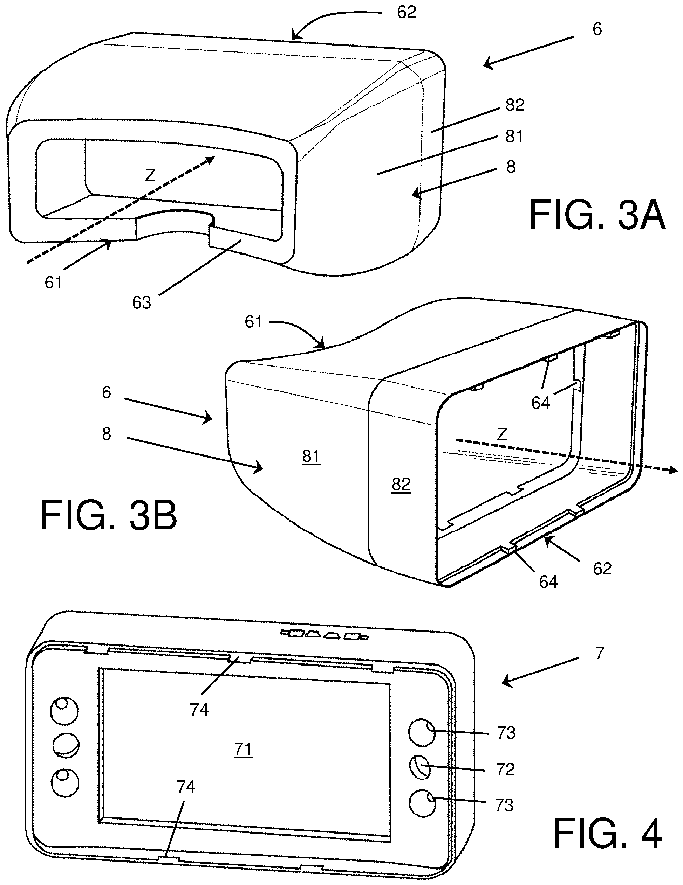

Le procédé selon l'invention peut être mis en oeuvre par un « ordinateur » au sens générique, par exemple par un système embarqué muni d'un circuit intégré du type FPGA qui a l'avantage de pouvoir être incorporé dans un élément portatif de puissance limitée vu que le procédé est particulièrement efficace. Un exemple préféré d'élément portatif est illustré en figure 4. Il s'agit d'une unité informatique 7 comprenant, outre le système embarqué susdit, un écran 71, deux caméras 72 et deux paires d'émetteurs infrarouge 73 agencés symétriquement de part et d'autre de l'écran 71. L'unité informatique 7 est configurée de sorte que l'écran 71 affiche des stimuli visuels, par exemple, des pixels en mouvement, alors que les caméras 72 captures des images d'un oeil ou des yeux d'un patient. Le système embarqué peut alors exécuter le procédé selon l'invention sur base de la séquence d'images capturées par une ou les deux caméras pour détecter les mouvements de l'œil ou des yeux, par exemple pour détecter des saccades oculaires, et optionnellement traiter les images extraites de la séquence d'images via l'étape (iii), par exemple, par segmentation comme décrit en détails dans l'exposé de l'invention.

L'unité informatique 7 est prévue pour être fixée de façon amovible en une deuxième extrémité 62 d'une chambre de vision 6 illustrée en figures 3A et 3B. La chambre de vision 6 comprend une paroi latérale 8 de forme approximativement parallélépipédique rectangle déformé coniquement et s'étendant autour d'un axe Z. Une première extrémité 61 de la chambre de vision 6 est opposé à la deuxième extrémité 62, l'axe Z étant dirigé de la première 61 vers la deuxième 62 extrémité. Un pourtour d'ouverture 63 est prévu au niveau de la première extrémité 61 pour être en contact avec une partie de tête du patient. Lorsque la chambre de vision 6 est utilisée, le front du patient s'appuie contre une partie supérieure du pourtour d'ouverture 63, et une partie du visage du patient entre ses yeux et sa bouche, au niveau de son nez, s'appuie contre une partie inférieure du pourtour d'ouverture 63 opposée à la partie supérieure, un renfoncement étant prévue dans la partie inférieure pour le nez du patient. Les yeux du patient sont orientés vers l'intérieur de la chambre de vision 6, selon l'axe Z, en direction de l'écran 71 lorsque l'unité informatique 7 est fixée en la deuxième extrémité 62. Pour la lisibilité des figures 3A et 3B, la chambre de vision 6 est représentée sans l'unité informatique 7.

La chambre de vision 6 fournit un environnement calibré adapté à la capture d'images de l'œil ou des yeux du patient par les caméra. Les émetteurs infrarouge 73 sont particulièrement utiles pour éclairer les yeux du patient aux fins de cette capture d'images vu que l'environnement de la chambre de vision 6 est mi-clos et sombre. Les images capturées sont ainsi de préférence des images infrarouge.

La paroi latérale 8 de la chambre de vision 6 est constituée d'une portion fixe 81 et d'une portion amovible 82 qui est fixée de façon amovible sur la portion fixe. Des éléments de fixation 64 amovibles sont prévus à cette fin au niveau extrémal de la portion fixe 81 opposé à la première extrémité 61 et aux extrémités la portion amovible 82. Ces éléments de fixation 64 consistent en des excroissances et des renfoncements (ou plus généralement des reliefs) qui coopèrent ensemble pour établir ladite fixation amovible entre la portion fixe 81 et la portion amovible 82, de façon semblable à l'emboitement de deux pièce de LEGO ®. Avantageusement, des éléments de fixation 74 semblables sont prévus sur l'unité informatique 7, par exemple autour de l'écran 71, pour coopérer avec les éléments de fixation 64 de la portion amovible 82 au niveau de la deuxième extrémité, et ainsi fixer de façon amovible l'unité informatique 7 sur la chambre de vision 6. Un tel système est très simple et pratique.

La portion amovible 82 rend l'ensemble dynamique et permet de modifier la distance séparant la première extrémité 61, donc les yeux du patient, et l'écran 71. Elle permet en particulier d'offrir deux longueurs de paroi latérale 8 mesurée le long de l'axe Z, fixes et calibrées. Une première longueur est obtenue en enlevant la portion amovible 82 et en fixant de façon amovible l'unité informatique 7 sur la portion fixe 81 via les éléments de fixation 74 de l'unité informatique et ceux 64 au niveau extrémal de la portion fixe 81. La deuxième longueur est obtenue avec la présence de la portion amovible 82, comme représenté en figures 3A et 3B. Bien entendu, d'autres portions amovibles semblables, mais n'ayant optionnellement pas toute la même longueur mesurée le long de l'axe Z, peuvent être prévues afin d'offrir plus que deux longueurs de paroi latérale 8 possibles, c'est-à-dire plusieurs distances possibles entre les yeux du patient et l'écran. Ces portions amovibles 82 peuvent être fixées successivement les unes aux autres de façon amovible sur et à partir de la portion fixe 81, grâce aux éléments de fixation 64, généralisant ainsi l'assemblage des figures 3A et 3B. L'unité informatique 7 est alors placée au niveau de la deuxième extrémité 62, la position de celle-ci dépendant du nombre de portions amovibles 82.

L'homme du métier comprendra que ces caractéristiques sur la chambre de vision 6 et sa fixation à l'unité informatique 7 peuvent également être considérées indépendamment du procédé selon l'invention et du fait que l'unité informatique 7 comprenne des moyens pour mettre en oeuvre ce procédé.

En résumé, l'invention concerne un procédé mis en oeuvre par ordinateur pour détecter un mouvement d'un corps à partir d'une séquence d'images de ce corps, en isolant (ou extrayant) les images 51 appartenant à des paires d'images en lesquelles une fonction dérivée 3 d'une mesure de similarité 2 de ces paires d'images est supérieur à un seuil 4 prédéterminé. De la sorte, le mouvement du corps est détecté sur ces images 51, lesquelles peuvent être ensuite traitées de façon plus précise aux fins d'étude du mouvement, sans nécessiter de traiter toutes les images de la séquence d'images.

La présente invention a été décrite ci-dessus en relation avec des modes de réalisations spécifiques, qui ont une valeur purement illustrative et ne doivent pas être considérés comme limitatifs. Il apparaîtra directement pour l'homme du métier que l'invention n'est pas limitée aux exemples illustrés ou décrits ci-dessus, et que sa portée est plus largement définie par les revendications ci-après introduites.

## Revendications

1. Procédé de détection d'un mouvement d'un corps à partir d'une séquence d'images de ce corps, le procédé étant mis en oeuvre par ordinateur et comprenant les étapes suivantes :

   (i) déterminer une mesure de similarité (2) de chaque paire d'images d'une séquence de paires d'images, la séquence de paires d'images étant induite par la séquence d'images et incluant chaque image de cette dernière ;
   (ii) déterminer une fonction dérivée (3) de la mesure de similarité (2) sur la séquence de paires d'images, la fonction dérivée (3) correspondant à une dérivée filtrée obtenue par combinaison avec un filtre passe-bas ;
   (iii) extraire au moins une image (51) de chaque paire d'images en laquelle la fonction dérivée (3) est supérieure à un seuil (4) strictement positif ;
   (iv) détecter le mouvement du corps sur base des images extraites à l'étape (iii).

2. Procédé selon la revendication 1, dans lequel la mesure de similarité (2) déterminée à l'étape (i) correspond à un coefficient de Bhattacharyya de distributions de probabilités discrètes associées aux images de la paire d'images et définies à partir de pixels de ces images.

3. Procédé selon la revendication 2, dans laquelle la distribution de probabilité discrète associée à chaque image est définie en un pixel de l'image comme un quotient d'un niveau de gris en ce pixel par une somme de niveaux de gris en chaque pixel de l'image.

4. Procédé selon l'une quelconque des revendications 1 à 3, dans lequel la dérivée filtrée correspond à une discrétisation bilinéaire d'une transformée de Laplace de fonction de transfert $H(s) = s\,(s\tau+1)^{-1}$ où $\tau$ est une constante.

5. Procédé selon l'une quelconque des revendications 1 à 4, dans lequel le seuil (4) est défini par un percentile d'une distribution de données obtenues par application de la fonction dérivée (3) à la séquence de paires d'images.

6. Procédé selon la revendication 5, dans lequel le percentile est choisi entre le quatre-vingtième et le nonante-neuvième percentile.

7. Procédé selon l'une quelconque des revendications 1 à 6, dans lequel l'étape (iii) comprend également une extraction périodique d'une image (52) de la séquence d'images.

8. Procédé selon l'une quelconque des revendications 1 à 7, dans lequel ladite au moins une image (51) extraite à l'étape (iii) comprend l'image de rang le plus élevé dans la séquence d'images parmi les deux images de la paire d'images.

9. Procédé selon l'une quelconque des revendications 1 à 8, dans lequel la séquence de paires d'images comprend chaque paire de deux images consécutives dans la séquence d'images.

10. Procédé selon l'une quelconque des revendications 1 à 8, dans lequel la séquence de paires d'images comprend chaque paire formée de la première image de la séquence d'images et d'une autre image de la séquence d'images.

11. Procédé selon l'une quelconque des revendications 1 à 10, comprenant une étape de segmentation des images extraites à l'étape (iii).

**12.** Procédé selon la revendication 11, dans lequel la segmentation de chaque image (51, 52) extraite à l'étape (iii) est définie par une fonction de segmentation de l'image, et dans lequel l'étape de segmentation est mise en oeuvre via les sous-étapes suivantes :

> (s1) déterminer une estimation de la fonction de segmentation de chaque image (51 52) extraite à l'étape (iii) ;
> (s2) déterminer récursivement la fonction de segmentation de chaque image (51, 52) extraite à l'étape (iii) à partir de son estimation.

**13.** Procédé selon la revendication 12, dans lequel l'estimation d'une fonction de segmentation d'une deuxième image est déterminée à la sous-étape (s1) par une composition d'un champ de vecteurs avec une fonction de segmentation d'une première image, et dans lequel

> - la première image précède la deuxième image dans la séquence d'images,
> - la fonction de segmentation de la première image est déterminée au préalable à la sous-étape (s2), et
> - le champ de vecteurs correspond en un déplacement de pixels entre la deuxième et la première images.

**14.** Méthode de suivi oculaire comprenant les étapes suivantes :

> (a) fournir une séquence d'images d'un oeil d'un patient ;
> (b) détecter un mouvement de l'œil à partir de la séquence d'images ; dans laquelle l'étape (b) est mise en oeuvre au moyen du procédé selon l'une quelconque des revendications 1 à 13.

**15.** Méthode selon la revendication 14, dans laquelle l'étape (b) est mise en oeuvre au moyen du procédé selon l'une quelconque des revendications 11 à 13, l'étape de segmentation étant mise en oeuvre au moins au voisinage d'une représentation de l'iris de l'œil sur chacune des images (51, 52) extraites à l'étape (iii), la méthode comprenant en outre les étapes suivantes :

> (c) déterminer une position d'un limbe de l'œil sur base de la segmentation des images (51, 52) extraites à l'étape (iii) ;
> (d) déterminer une position de l'œil sur base de la position du limbe de l'œil déterminé à l'étape (c).

**16.** Utilisation de la méthode selon la revendication 14 ou 15, pour détecter une saccade de l'œil.

**17.** Système informatique de traitement de données comprenant des moyens configurés pour mettre en oeuvre le procédé selon l'une quelconque des revendications 1 à 13.

**18.** Système informatique selon la revendication 17, consistant en un système embarqué muni d'un circuit intégré du type FPGA.

**19.** Dispositif de détection d'un mouvement d'un oeil d'un patient comprenant une chambre de vision (6) pour le patient comprenant, en une première extrémité (61), un pourtour d'ouverture (63) apte à être en contact avec une partie de tête du patient, et en une deuxième extrémité (62), un écran (71) d'affichage de stimuli visuels ; l'écran (71) étant intégré dans une unité informatique (7) comprenant :

> - le système informatique selon la revendication 18 ;
> - au moins une caméra (72) d'acquisition d'images de l'œil dirigée vers la première extrémité (61) et couplée électroniquement au système informatique pour lui transmettre une séquence d'images de l'œil.

**20.** Dispositif selon la revendication 19, dans lequel la chambre de vision (6) comprend une paroi latérale (8) s'étendant autour d'un axe (Z), entre les première (61) et deuxième (62) extrémités, une distance entre celles-ci étant réglable à une longueur fixe de la paroi latérale (8) mesurée le long de l'axe (Z) parmi différentes longueurs possibles, les différentes longueurs possibles étant obtenues par fixations amovibles successives d'une ou plusieurs portions amovibles (82) de la paroi latérale (8) à une portion fixe (81) de la paroi latérale (8) s'étendant à partir de la première extrémité (61).

**21.** Dispositif selon la revendication 19 ou 20, dans lequel l'unité informatique (7) comprend en outre une horloge centrale configurée pour commander :

- un affichage des stimuli visuels, au moyen de l'écran (71), par un rafraîchissement de pixels de l'écran (71) à une première fréquence ;
- une acquisition des images de l'œil, au moyen de la caméra (72), à une deuxième fréquence ;

les première et deuxième fréquences étant égales ou multiples l'une de l'autre par un nombre naturel non nul.

22. Programme d'ordinateur comprenant des instructions qui,
lorsque le programme d'ordinateur est exécuté par un ordinateur, conduisent celui-ci à mettre en oeuvre le procédé selon l'une quelconque des revendications 1 à 13.

23. Support informatique lisible par un ordinateur et sur lequel est enregistré un programme d'ordinateur selon la revendication 22.

**Patentansprüche**

1. Verfahren zum Erkennen einer Bewegung eines Körpers aus einer Abfolge von Bildern dieses Körpers, wobei das Verfahren durch einen Computer ausgeführt wird und die folgenden Schritte umfasst:

(i) Bestimmen einer Ähnlichkeitsmessung (2) eines jeden Bildpaares einer Abfolge von Bildpaaren, wobei die Abfolge von Bildpaaren durch die Abfolge von Bildern induziert wird und jedes Bild dieser letzteren beinhaltet;
(ii) bestimmen einer von der Ähnlichkeitmessung (2) an der Abfolge von Bildpaaren abgeleiteten Funktion (3), wobei die abgeleitete Funktion (3) einer gefilterten Ableitung entspricht, die durch Kombination mit einem Tiefpassfilter erhalten wird;
(iii) Extrahieren mindestens eines Bildes (51) eines jeden Bildpaares, in dem die abgeleitete Funktion (3) größer als eine streng positive Schwelle (4) ist;
(iv) Erkennen der Bewegung des Körpers auf Grundlage der im Schritt (iii) extrahierten Bilder.

2. Verfahren nach Anspruch 1, wobei die im Schritt (i) bestimmte Ähnlichkeitsmessung (2) einem Bhattacharyya Koeffizienten diskreter Wahrscheinlichkeitsverteilungen entspricht, die den Bildern des Paares von Bildern zugeordnet sind, und aus Pixeln dieser Bilder definiert sind.

3. Verfahren nach Anspruch 2, wobei die diskrete Wahrscheinlichkeitsverteilung, die einem jeden Bild zu geordnet ist, in einem Pixel des Bildes als ein Quotient einer Graustufe in diesem Pixel durch eine Summe von Graustufen in jedem Pixel des Bildes definiert ist.

4. Verfahren nach einem der Ansprüche 1 bis 3, wobei die gefilterte Ableitung einer bilinearen Diskretisierung einer Laplace-Transformation einer Übertragungsfunktion $H(s) = s\,(s\tau +1)^{-1}$ entspricht, wobei $\tau$ eine Konstante ist.

5. Verfahren nach einem der Ansprüche 1 bis 4, wobei die Schwelle (4) durch ein Perzentil einer Verteilung von Daten definiert ist, die durch Anwenden der abgeleiteten Funktion (3) auf die Abfolge von Bildpaare, erhalten werden.

6. Verfahren nach Anspruch 5, wobei das Perzentil zwischen dem 80. und dem 99. Perzentil ausgewählt wird.

7. Verfahren nach einem der Ansprüche 1 bis 6, wobei der Schritt (iii) auch eine periodische Extraktion eines Bildes (52) der Abfolge von Bildern umfasst.

8. Verfahren nach einem der Ansprüche 1 bis 7, wobei das mindestens eine im Schritt (iii) extrahierte Bild (51) das Bild mit dem höchsten Rang in der Abfolge von Bildern unter den beiden Bildern des Bildpaares umfasst.

9. Verfahren nach einem der Ansprüche 1 bis 8, wobei die Abfolge von Bildpaaren jedes Paar zweier aufeinanderfolgender Bilder in der Abfolge von Bildern umfasst.

10. Verfahren nach einem der Ansprüche 1 bis 8, wobei die Abfolge von Bildpaaren jedes Paar umfasst, das aus dem ersten Bild der Abfolge von Bildern und einem anderen Bild der Abfolge von Bildern gebildet wird.

11. Verfahren nach einem der Ansprüche 1 bis 10, umfassend einen Schritt zum Segmentieren der im Schritt (iii) extrahierten Bilder.

**12.** Verfahren nach Anspruch 11, wobei die Segmentierung eines jeden im Schritt (iii) extrahierten Bildes (51,52) durch eine Segmentierungsfunktion des Bildes definiert wird, und wobei der Segmentierungsschritt anhand der folgenden Teilschritte ausgeführt wird:

(s1) Bestimmen einer Schätzung der Segmentierungsfunktion eines jeden im Schritt (iii) extrahierten Bildes (51 52);
(s2) rekursiv Bestimmen der Segmentierungsfunktion eines jeden im Schritt (iii) extrahierten Bildes (51,52) aus seiner Schätzung.

**13.** Verfahren nach Anspruch 12, wobei die Schätzung einer Segmentierungsfunktion eines zweiten Bildes im Teilschritt (s1) durch eine Zusammensetzung eines Vektorfeldes mit einer Segmentierungsfunktion eines ersten Bildes bestimmt wird, und wobei

- das erste Bild dem zweiten Bild in der Abfolge von Bildern vorangeht,
- die Segmentierungsfunktion des ersten Bildes vor dem Teilschritt (s2) bestimmt wird, und
- das Vektorfeld einer Pixelverschiebung zwischen dem zweiten und dem ersten Bild entspricht.

**14.** Augenverfolgungsverfahren, das die folgenden Schritte umfasst:

(a) Bereitstellen einer Abfolge von Bildern eines Auges eines Patienten;
(b) Erkennen einer Bewegung des Auges aus der Abfolge von Bildern;

wobei Schritt (b) anhand des Verfahrens nach einem der Ansprüche 1 bis 13 implementiert wird.

**15.** Verfahren nach Anspruch 14, wobei der Schritt (b) anhand des Verfahrens nach einem der Ansprüche 11 bis 13 ausgeführt wird, der Segmentierungsschritt mindestens in der Umgebung einer Darstellung der Iris des Auges auf jedem der im Schritt (iii) extrahierten Bilder (51,52) ausgeführt wird, wobei das Verfahren weiter die folgenden Schritte umfasst:

(c) Bestimmen einer Position eines Limbus des Auges auf Grundlage der Segmentierungen der im Schritt (iii) extrahierten Bilder;
(d) Bestimmen einer Position des Auges auf Grundlage der in Schritt (c) bestimmten Position des Limbus des Auges.

**16.** Verwendung des Verfahrens nach Anspruch 14 oder 15 zum Erkennen eines Rucks des Auges.

**17.** Informationstechnisches Datenverarbeitungssystem, das Mittel umfasst, die so konfiguriert sind, um das Verfahren nach einem der Ansprüche 1 bis 13 auszuführen.

**18.** Informationstechnisches System nach Anspruch 17, das aus einem eingebauten System besteht, das mit einer integrierten Schaltung vom Typ FPGA versehen ist.

**19.** Vorrichtung zum Erkennen einer Bewegung eines Auges eines Patienten, die eine Visionskammer (6) für den Patienten umfasst, die an einem ersten Ende (61) einen Öffnungsumfang (63), der imstande ist, in Kontakt mit einem Kopfteil des Patienten zu sein, und an einem zweiten Ende (62) einen Anzeigebildschirm (71) für optische Reize umfasst; wobei der Bildschirm (71) in einer informationstechnischen Einheit (7) integriert ist, die umfasst:

- das informationstechnische System nach Anspruch 18;
- mindestens eine Kamera (72) zum Erfassen von Bildern des Auges, die zum ersten Ende (61) gerichtet ist, und elektronisch mit dem informationstechnischen System gekoppelt ist, um diesem eine Abfolge von Bildern des Auges zu übertragen.

**20.** Vorrichtung nach Anspruch 19, wobei die Visionskammer (6) eine Seitenwand (8) umfasst, die sich um eine Achse (Z) herum zwischen dem ersten (61) und zweiten (62) Ende erstreckt, wobei ein Abstand zwischen diesen auf eine feste Länge der Seitenwand (8) einstellbar ist, die entlang der Achse (Z) aus verschiedenen möglichen Längen gemessen wird, wobei die verschiedenen möglichen Längen durch aufeinanderfolgende entfernbare Befestigungen eines oder mehrerer entfernbarer Abschnitte (82) der Seitenwand (8) in einem festen Abschnitt (81) der Seitenwand (8) erhalten werden, der sich aus dem ersten Ende (61) erstreckt.

**21.** Vorrichtung nach Anspruch 19 oder 20, wobei die informationstechnische Einheit (7) weiter eine Hauptuhr umfasst, die konfiguriert ist, um zu steuern:

 - eine Anzeige der optischen Reize anhand des Bildschirms (71), durch Auffrischen von Pixeln des Bildschirms (71) mit einer ersten Frequenz;
 - eine Erfassung der Bilder des Auges anhand der Kamera (72) mit einer zweiten Frequenz;

wobei die erste und zweite Frequenz gleich oder Vielfache voneinander um eine natürliche Zahl ungleich null sind.

**22.** Computerprogramm, das Anweisungen umfasst, die, wenn das Computerprogramm von einem Computer ausgeführt wird, diesen veranlassen, das Verfahren nach einem der Ansprüche 1 bis 13 auszuführen.

**23.** Informationstechnischer Träger, der durch einen Computer lesbar ist, und auf dem ein Computerprogramm nach Anspruch 22 gespeichert ist.

**Claims**

**1.** A method for detecting a movement of a body from a sequence of images of this body, the method being computer-implemented and comprising the following steps:

 (i) determining a similarity measure (2) of each image pair of a sequence of image pairs, the sequence of image pairs being induced by the sequence of images and including each image thereof;
 (ii) determining a derivative function (3) of the similarity measure (2) on the sequence of image pairs, the derivative function (3) corresponding to a filtered derivative obtained by combination with a low-pass filter;
 (iii) extracting at least one image (51) from each image pair in which the derivative function (3) is greater than a positive threshold (4);
 (iv) detecting the movement of the body on the basis of the images extracted in the step (iii).

**2.** The method according to claim 1, wherein the similarity measure (2) determined in the step (i) corresponds to a Bhattacharyya coefficient of discrete probability distributions associated with the images of the image pair and defined from pixels of these images.

**3.** The method according to claim 2, wherein the discrete probability distribution associated with each image is defined at a pixel of the image as a quotient of a grey level at that pixel by a sum of grey levels at each pixel of the image.

**4.** The method according to any one of claims 1 to 3, wherein the filtered derivative corresponds to a bilinear discretisation of a transfer function Laplace transform $H(s) = s\,(s\tau+1)^{-1}$ where $\tau$ is a constant.

**5.** The method according to any one of claims 1 to 4, wherein the threshold (4) is defined by a percentile of a distribution of data obtained by applying the derivative function (3) to the sequence of image pairs.

**6.** The method according to claim 5, wherein the percentile is chosen between the eightieth and the ninety-ninth percentile.

**7.** The method according to any one of claims 1 to 6, wherein the step (iii) also comprises periodically extracting an image (52) from the sequence of images.

**8.** The method according to any one of claims 1 to 7, wherein said at least one image (51) extracted in the step (iii) comprises the image of highest rank in the sequence of images among the two images of the image pair.

**9.** The method according to any one of claims 1 to 8, wherein the sequence of image pairs comprises each pair of two consecutive images in the sequence of images.

**10.** The method according to any one of claims 1 to 8, wherein the sequence of image pairs comprises each pair formed of the first image of the sequence of images and a further image of the sequence of images.

**11.** The method according to any one of claims 1 to 10, comprising a segmentation step of the images extracted in the step (iii).

12. The method according to claim 11, wherein the segmentation of each image (51, 52) extracted in the step (iii) is defined by a segmentation function of the image, and wherein the segmentation step is implemented via the following sub-steps:

(s1) determining an estimate of the segmentation function of each image (51 52) extracted in the step (iii);
(s2) recursively determining the segmentation function of each image (51, 52) extracted in the step (iii) on the basis of its estimate.

13. The method according to claim 12, wherein the estimate of a segmentation function of a second image is determined in the substep (s1) by a composition of a vector field with a segmentation function of a first image, and wherein

- the first image precedes the second image in the sequence of images,
- the segmentation function of the first image is determined beforehand in the sub-step (s2), and
- the vector field corresponds to a displacement of pixels between the second and first images.

14. An eye-tracking method comprising the following steps:

(a) providing a sequence of images of an eye of a patient;
(b) detecting a movement of the eye from the sequence of images;

wherein the step (b) is carried out by means of the method according to any one of claims 1 to 13.

15. The method according to claim 14, wherein the step (b) is implemented by means of the method according to any one of claims 11 to 13, the segmentation step being implemented at least in the vicinity of a representation of the iris of the eye on each of the images (51, 52) extracted in the step (iii), the method further comprising the following steps:

(c) determining a position of a limbus of the eye on the basis of the segmentation of the images (51, 52) extracted in the step (iii);
(d) determining a position of the eye on the basis of the position of the limbus of the eye determined in the step (c).

16. Use of the method according to claim 14 or 15, to detect a saccade of the eye.

17. A data processing computer system comprising means configured to implement the method according to any one of claims 1 to 13.

18. The computer system according to claim 17, consisting of an embedded system equipped with an FPGA-type integrated circuit.

19. A device for detecting a movement of an eye of a patient comprising a viewing chamber (6) for the patient comprising, at a first end (61), an opening periphery (63) capable of being in contact with a head part of the patient, and at a second end (62), a screen (71) for displaying visual stimuli; the screen (71) being integrated into a computer unit (7) comprising:

- the computer system according to claim 18;
- at least one camera (72) for acquiring images of the eye directed towards the first end (61) and electronically coupled to the computer system in order to transmit to the latter a sequence of images of the eye.

20. The device according to claim 19, wherein the viewing chamber (6) comprises a side wall (8) extending about an axis (Z), between the first (61) and the second (62) ends, a distance therebetween being adjustable to a fixed length of the side wall (8) measured along the axis (Z) from among different possible lengths, the different possible lengths being obtained by successive removable attachments of one or more removable portions (82) of the side wall (8) to a fixed portion (81) of the side wall (8) extending from the first end (61).

21. The device according to claim 19 or 20, wherein the computer unit (7) further comprises a central clock configured to control:

- a display of the visual stimuli, by means of the screen (71), by refreshing pixels of the screen (71) at a first frequency;
- an acquisition of images of the eye, by means of the camera (72), at a second frequency;

the first and second frequencies being equal to or multiples of each other by a non-zero natural number.

22. A computer program comprising instructions which,

when the computer programme is run by a computer,
lead the latter to implement the method according to any one of claims 1 to 13.

23. A computer-readable medium on which a computer program according to claim 22 is recorded.

FIG. 1

FIG. 2

EP 4 402 648 B1

FIG. 3A

FIG. 3B

FIG. 4

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- Eye Blink Detection Using Local Binary Patterns. **K. MALIK ; B. SMOLKA.** ICMCS. IEEE, 2014